# FASCICULE DE BREVET EUROPEEN

(11) **EP 2 683 340 B1**
(45) Date de publication et mention de la délivrance du brevet: **06.04.2016**
(21) Numéro de dépôt: 11815764.3
(22) Date de dépôt: 30.12.2011
(51) Int. Cl.: A61F 5/058, A61F 5/56

(54) **DISPOSITIF BUCCAL**
ORALE VORRICHTUNG
ORAL DEVICE

(30) Priorité: 08.03.2011 FR 1100686
(43) Date de publication de la demande: 15.01.2014
(73) Titulaire: Fellus, Patrick André, 75006 Paris (FR)
(72) Inventeur: Fellus, Patrick André, 75006 Paris (FR)
(74) Mandataire: Palacci, Jeremie
(86) Numéro de dépôt international: PCT/FR2011/000685
(87) Numéro de publication internationale: WO 2012/120203

(56) Documents cités:
- EP-A1- 2 189 131
- DE-U1- 8 323 817
- DE-U1-202009 003 914
- US-A- 6 129 084
- US-A1- 2008 149 110

## Description

L'invention concerne le domaine de la croissance faciale infantile.

La croissance faciale est un domaine faisant l'objet de nombreuses recherches et qui est en perpétuelle évolution. Au cours de ces évolutions, il arrive que des dogmes soient progressivement modifiés et/ou bouleversés.

Parmi les dogmes bien établis, on compte la notion qu'il vaut mieux attendre que la croissance maxillo-mandibulaire soit terminée, ou que toutes les dents définitives aient évolué avant d'opérer un traitement des dysmorphoses maxillo-mandibulaires, car la taille de la mandibule est prédéterminée, et il n'est pas possible de l'influencer.

Les travaux de recherche clinique effectués par le Demandeur au cours des 25 dernières années ont renversé ce dogme, et ont montré tout l'intérêt de l'orthodontie précoce dans une pratique visant la prévention.

Ces travaux ne visent pas à traiter tous les enfants en denture lactéale, mais à corriger de manière précoce un certain nombre de dysmorphoses squelettiques avant l'âge classique de traitement orthodontique.

Récemment, le Demandeur a identifié un ensemble de conditions maxillo-mandibulaires liées à une déficience dans l'acquisition de la déglutition de type « sujet denté » (également appelée déglutition adulte). En pratique, cela signifie que certains enfants conservent trop longtemps une praxie de succion-déglutition (également appelée déglutition infantile ou déglutition primaire).

À ce jour, ces travaux restent précurseurs dans ce domaine et il n'existe pas de méthode ni de dispositif adaptés à leur mise en oeuvre.

L'invention vient améliorer la situation.

À cet effet, le Demandeur propose un dispositif buccal, agencé pour être porté en bouche par une personne, et pour solliciter le nerf trijumeau lors de la déglutition. Avantageusement ce dispositif peut comprendre une portion supérieure présentant une forme sensiblement en gouttière, et propre à se loger entre une lèvre supérieure et une arcade dentaire, une portion inférieure présentant une forme sensiblement en gouttière, et propre à se loger entre une lèvre inférieure et une arcade dentaire, ainsi qu'une ouverture entre la portion supérieure et la portion inférieure. La portion supérieure et la portion inférieure sont reliées entre elles à des extrémités respectives, de sorte que, lorsque le dispositif est reçu dans une bouche, la musculature labiale est sensiblement au repos, et au moins une partie de l'ouverture reste libre. L'ouverture présente une hauteur maximale d'environ 1,5 cm.

Le Demandeur propose également un procédé d'apprentissage d'une praxie, comprenant le port d'un dispositif buccal par une personne, agencé pour solliciter le nerf trijumeau lors de la déglutition. Avantageusement ce procédé peut comprendre la mise en place du dispositif au moins en partie entre les lèvres et l'arcade dentaire de la personne, et le maintien de la musculature labiale sensiblement au repos ainsi que le maintien d'une ouverture entre les lèvres pendant la durée du port.

Ce dispositif et ce procédé présentent de nombreux avantages qui seront explicités plus bas.

D'autres caractéristiques et avantages de l'invention apparaîtront mieux à la lecture de la description qui suit, tirée d'exemples donnés à titre illustratif et non limitatif, tirés des dessins sur lesquels :
- la figure 1 représente une vue en perspective de trois-quarts d'un dispositif selon l'invention,
- la figure 2 représente une vue de derrière du dispositif de la figure 1,
- la figure 3 représente une vue de face du dispositif de la figure 1,
- la figure 4 représente une vue du côté gauche du dispositif de la figure 1,
- la figure 5 représente une vue du côté droit du dispositif de la figure 1,
- la figure 6 représente une vue en perspective de trois-quarts d'un autre mode de réalisation d'un dispositif,
- la figure 7 représente une vue de derrière du dispositif de la figure 6,
- la figure 8 représente une vue de face du dispositif de la figure 6,
- la figure 9 représente une vue du côté gauche du dispositif de la figure 6, et
- la figure 10 représente une vue du côté droit du dispositif de la figure 6.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Le squelette de l'étage moyen du visage d'un enfant connaît une croissance qui est conditionnée par son environnement fonctionnel. Dans le cas de la cavité orbitaire, c'est l'oeil qui joue le rôle de conformateur. Dans le cas de la boîte crânienne, ce sera le cerveau. En ce qui concerne la cavité buccale, c'est la langue qui joue le rôle de conformateur, aussitôt que la déglutition de type sujet denté a été acquise.

En effet, la déglutition de type sujet denté implique que le dôme lingual vienne s'appliquer contre la voûte palatine, tant lors des mouvements de déglutition que dans la posture habituelle, ce qui stimule la croissance transversale des maxillaires.

Traditionnellement, les enfants acquièrent la déglutition de type sujet denté entre l'âge de trois ans et l'âge de cinq ans, âges auxquels la denture lactéale est constituée et où les habitudes masticatoires deviennent acquises.

Chez les enfants qui n'acquièrent pas la déglutition de type sujet denté, la succion-déglutition persiste. Mais les forces musculaires qui sont mises en oeuvre avec la succion-déglutition entraînent des troubles de la croissance faciale.

En effet, dans la succion-déglutition, les arcades dentaires ne sont pas en contact lors des mouvements de déglutition, et la langue s'interpose entre le maxillaire et la mandibule afin de trouver un contact avec la muqueuse labiale ou jugale.

De ce fait, le dôme lingual ne vient plus stimuler la croissance de la voute palatine qui est au contraire entravée par la dépression des buccinateurs qui découle des mouvements de succion.

De plus, la contraction de la musculature labiale inférieure et mentonnière maintient la mandibule dans une position rétrusive ce qui est caractéristique d'une rétromandibulie fonctionnelle qui deviendra, si elle n'est pas corrigée une rétrognathie mandibulaire chez l'adolescent. Similairement, le maintien d'une position basse habituelle de la langue entraîne une avancée mandibulaire et peut alors transformer le proglissement initial en prognathie.

L'absence de contact entre la langue et la voûte palatine, conjuguée à une fonction incisive inversée, associera une hypoplasie de l'étage moyen à la dysmorphose mandibulaire.

D'autres conditions de développement défavorables de la mandibule des enfants peuvent être reliées à un déficit d'acquisition de la déglutition de type sujet denté.

En plus des problèmes physiologiques qui les accompagnent, ces dysmorphoses ont souvent des conséquences importantes d'un point de vue psychologique, du fait des problèmes esthétiques qui leur sont associés, ce qui retarde souvent l'intégration sociale de l'enfant.

À l'inverse, l'acquisition de la déglutition de type sujet denté favorise l'apprentissage de la respiration nasale, ainsi que l'arrêt de la succion du pouce de manière plus précoce.

Tous ces éléments participent à l'acquisition d'une croissance eumorphique.

Les travaux du Demandeur ont montré que le passage de la succion-déglutition à la déglutition de type sujet denté est conditionné par l'établissement de nouvelles images motrices. En effet, les mouvements habituels effectués sans contrôle conscient sont exécutés selon une séquence d'images motrices aussi proches les unes des autres que les images d'une séquence filmée. Ces nouvelles images motrices peuvent être acquises par apprentissage, et enrichir le schéma corporel dans la zone orale.

Dans le cadre présent, ce schéma repose sur la déconnexion de la syncinésie lèvre/langue et l'élévation du dôme lingual. Concrètement, il s'agit d'apprendre à avaler sans faire appel aux muscles des joues et des lèvres. La déconnexion de la syncinésie lèvre/langue et l'élévation du dôme lingual se traduit principalement par le passage d'une sollicitation du nerf facial (VII) dans le cadre de la succion-déglutition vers une sollicitation du nerf trijumeau (V) dans le cadre de la déglutition de type sujet denté.

Il s'agit donc d'apprendre à l'enfant à abandonner la succion-déglutition et à acquérir une nouvelle praxie de déglutition de type sujet denté. L'automatisation de ce type de déglutition, répété une fois par minute, modifiera l'équilibre des forces musculaires et la langue jouera ainsi par les 17 muscles la constituant le rôle de matrice fonctionnelle.

Les travaux du Demandeur l'ont amené à constater que sans ce nouvel équilibre musculaire, les traitements mécaniques classiques durent plus longtemps et les résultats ne seront pas toujours stables.

Actuellement, il n'existe pas de méthode ou de dispositif efficace qui permette de réaliser l'apprentissage de la transition succion-déglutition vers déglutition de type sujet denté.

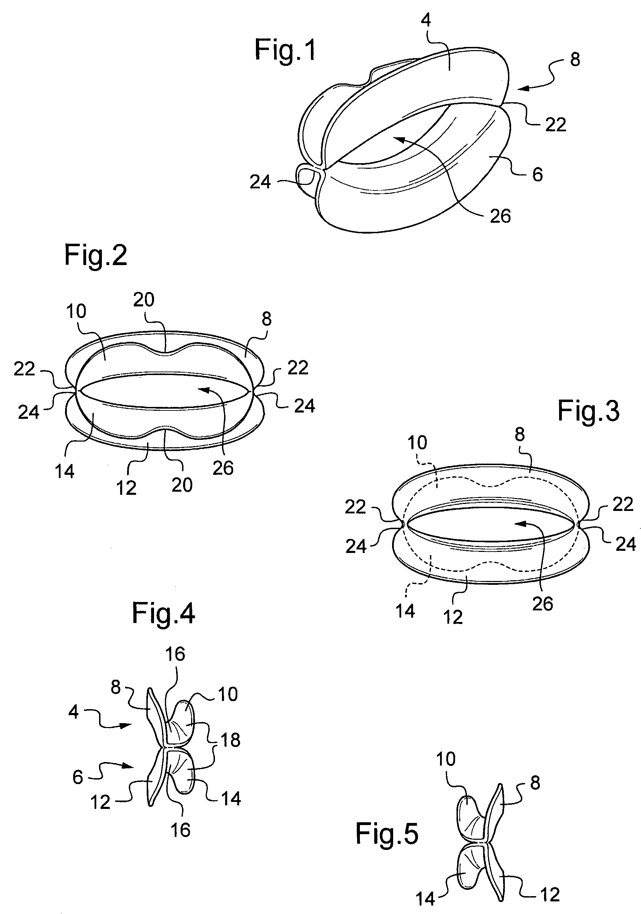

En réponse à ces problèmes, le Demandeur a conçu le dispositif représenté sur la figure 1.

Comme on peut le voir sur cette figure, le dispositif buccal 2 comprend une portion supérieure 4 et une portion inférieure 6.

Comme on peut le voir sur les figures 4 et 5, la portion supérieure 4 et la portion inférieure 6 présentent chacune une forme sensiblement en gouttière. Ainsi, la portion supérieure 4 présente une section avec une branche 8, et une branche 10 sensiblement perpendiculaire à la branche 8. La portion inférieure 6 présente une section avec une branche 12 et une branche 14 sensiblement perpendiculaire à la branche 12.

La branche 10 présente une forme courbée propre à recevoir la lèvre supérieure, et à se loger entre la face intérieure de la lèvre supérieure et l'arcade dentaire supérieure. La branche 14 présente une forme courbée propre à recevoir la lèvre inférieure, et à se loger entre la face intérieure de la lèvre inférieure et l'arcade dentaire inférieure. La face extérieure de la lèvre supérieure et la face extérieure de la lèvre inférieure viennent se loger au contact respectivement de la branche 8 et de la branche 12.

La branche 10 et la branche 14 qui sont de forme courbées comprennent deux parties :
- une première partie 16 sensiblement plane sur laquelle vient s'appuyer la face inférieure de la lèvre supérieure et respectivement la face supérieure de la lèvre inférieure,
- une deuxième partie 18 sensiblement bombée qui vient se loger entre la face intérieure des lèvres et l'arcade dentaire.

La première partie 16 est sensiblement plane. La première partie 16 forme un angle d'environ 90° avec la branche 8 (respectivement la branche 12). L'extrémité de la première partie 16 est courbée, de sorte que la deuxième partie 18 forme un angle avec la première partie 16. La forme bombée de deuxième partie 18 est agencée de manière à épouser la forme de l'arcade dentaire.

Comme on peut le voir sur la figure 2, les branches 10 et 14 présentent une découpe 20 sensiblement au milieu de la deuxième partie 18. La découpe 20 est sensiblement arrondie, de sorte qu'elle correspond sensiblement au frein de chaque lèvre, et facilite la mise en place du dispositif 2 dans la bouche.

Ainsi, le dispositif 2 vient se loger dans la bouche, et y est maintenu sans effort musculaire des lèvres, ce qui offre un bon confort d'utilisation.

Comme cela apparaît plus clairement sur les figures 1, 2 et 3, la portion supérieure 4 et la portion inférieure 6 sont reliées entre elles à des extrémités respectives 22 et 24 des portions 18 des branches 10 et 14 de sorte qu'elles définissent entre elles une ouverture 26 de forme sensiblement ovale. Cette ouverture 26 est dimensionnée de sorte qu'elle reste toujours au moins partiellement libre lorsqu'une personne porte le dispositif 2. Pour cela, elle est conçue de manière à être plus épaisse que la partie antérieure de la langue.

Dans l'exemple décrit ici, la portion supérieure 4 et la portion inférieure 6 sont collées au niveau des extrémités 22 et 24. En variante, ces extrémités comprennent des éléments de verrouillage coopérants, permettant de détacher la portion supérieure 4 de la portion supérieure 6 lorsque le disposition buccal 2 n'est pas porté, et de les relier simplement, par exemple par clipsage ou tout autre moyen approprié, pour le port. Dans une autre variante, ces extrémités sont soudées.

Dans l'exemple décrit ici, le dispositif 2 présente une largeur totale de 5 cm et l'ouverture 26 présente une largeur d'environ 3,5 cm. Les branches 8 et 12 présentent une hauteur d'environ 1 cm et les branches 10 et 14 présentent une longueur d'environ 7,5 mm, la partie 18 recourbée de ces branches remontant d'environ 4 mm. L'ouverture 26 présente une hauteur maximale d'environ 1,5 cm.

D'une manière générale, la largeur du dispositif 2 est prévue pour correspondre sensiblement à l'espace entre les commissures des lèvres de la personne. En particulier, cette largeur pourra être prévue légèrement supérieure pour faciliter le maintien sans solliciter les muscles. L'ouverture 26 est prévue pour être plus importante que l'apex lingual. Afin d'accommoder toutes les bouches, plusieurs tailles pourront être prévues pour le dispositif, par exemple trois tailles. En variante, ces différentes tailles pourront être obtenues en utilisant des éléments de verrouillage coopérants de tailles différentes, ou télescopiques.

La portion supérieure 4 et la portion inférieure 6 présentent chacune un plan de symétrie, qui est sensiblement perpendiculaire au plan des figures 2 et 3, au niveau de la découpe 20. Ce plan de symétrie reflète la symétrie de la bouche humaine. En outre, le dispositif 2 pourra dans certains cas présenter un plan de symétrie supplémentaire, également perpendiculaire au plan des figures 2 et 3, mais cette fois au niveau de l'ouverture 26, de sorte que la portion supérieure 4 et la portion inférieure 6 sont symétriques l'une de l'autre par rapport à ce plan.

En variante, la portion supérieure et la portion inférieure peuvent présenter chacune une largeur de gouttière plus importante à l'opposé de l'ouverture qu'à proximité de l'ouverture, pour permettre un meilleur maintien et pour faciliter la mise en place. Autrement dit, le profil des portions supérieure et inférieure peut être évasé.

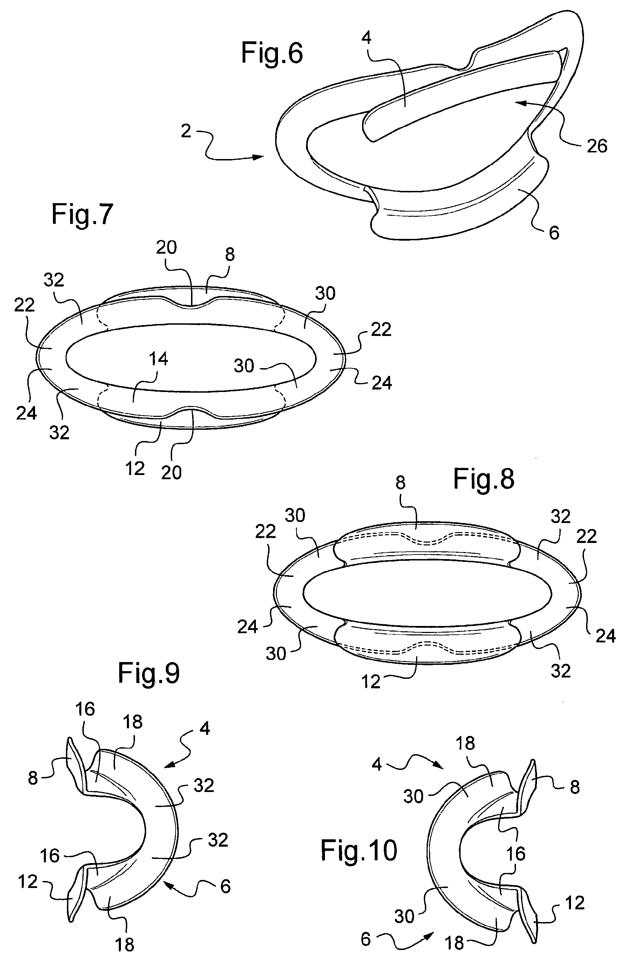

Les figures 6 à 10 représentent un dispositif buccal dont la hauteur de l'ouverture 26 est supérieure à celle revendiquée. Dans cette variante, le dispositif buccal 2 est monobloc, et est par exemple réalisé par moulage ou par tout autre moyen adapté. Ainsi, les branches 10 et 14 comprennent chacune une troisième partie 30 et une quatrième partie 32.

Comme on peut le voir sur les figures 7 et 8, les troisièmes parties 30 et les quatrièmes parties 32 sont sensiblement symétriques l'une de l'autre par rapport à un plan sensiblement perpendiculaire au plan des figures 7 et 8, au niveau de la découpe 20, et sont en prolongement des deuxièmes parties 18, de part et d'autre de celles-ci.

La troisième partie 30 de la branche 10 est reliée à la troisième partie 30 de la branche 14, et la quatrième partie 32 de la branche 10 est reliée à la quatrième partie 32 de la branche 14.

Dans cette variante, le dispositif 2 présente une largeur totale de 8,5 cm. L'ouverture 26 est de forme générale oblongue, et présente une hauteur maximale d'environ 2 cm, soit supérieure à celle revendiquée, et une largeur maximale d'environ 7,5 cm. Les branches 8 et 12 présentent une hauteur d'environ 1 cm et une largeur d'environ 3 cm. La partie 16 des branches 10 et 14 s'étend sur une profondeur d'environ 7,5 mm, et la partie 18 recourbée remonte d'environ 4 mm.

Selon la taille choisie pour le dispositif 2, c'est-à-dire en fonction des dimensions de la bouche de l'enfant, ces dimensions pourront varier. Ainsi, la largeur totale du dispositif pourra être comprise entre 3 cm et 10 cm, l'ouverture 26 pourra avoir une largeur maximale comprise entre 2 cm et 9 cm, et une hauteur maximale comprise entre 3 mm et 4,5 cm. Les branches 8 et 12 pourront présenter une hauteur comprise entre 5 mm et 2 cm, et une largeur comprise entre 2 cm et 5 cm, soit une valeur proche de la distance entre les commissures. La partie 16 des branches 10 et 14 pourra s'étendre sur une profondeur comprise entre 3 mm et 3 cm, et la partie 18 recourbée pourra remonter d'environ 2 mm à 1 cm. Ces dimensions peuvent être appliquées au mode de réalisation des figures 1 à 5.

Avantageusement, la portion de liaison des parties 30 et 32 épouse sensiblement le contour intérieur des lèvres, et la liaison à lieu au niveau des modioli de la commissure des joues, contrariant ainsi la contraction des muscles à ce niveau.

Il ressort des deux modes de réalisation décrits que :
- les portions supérieure 4 et inférieure 6 ont une forme sensiblement en gouttière,
- le reste du dispositif est disposé de manière postérieure à un plan défini par un des bords de ces gouttières, au-delà de la portion reliant les bords de ces gouttières.

Par une forme sensiblement en gouttière, on entend le fait que l'ensemble branches 8 et 10 et l'ensemble branches 12 et 14 logent les lèvres à la manière d'une gouttière. Ainsi, les lèvres sont sensiblement au repos et leur contraction est contrariée.

Cependant, il serait possible d'évider en partie ou grandement certaines de ces branches, tout en conservant cette fonction, par exemple en retirant de la matière, ou en réalisant ces branches sous forme de grillage. De telles réalisations rentrent dans la portée de l'invention.

Les travaux du Demandeur ont montré que le port du dispositif 2 par une personne, et en particulier un enfant, n'est que peu gênant, mais permet en revanche une acquisition spontanée de la déglutition de type sujet denté.

En effet, le dispositif 2 maintient les lèvres inférieure et supérieure à l'écart l'une de l'autre, empêchant la réalisation d'un joint d'étanchéité entre les deux lèvres par le biais de l'ouverture 26, ce qui empêche de créer une dépression à l'intérieur de la cavité buccale par succion. En réaction, la personne ne pourra qu'élever la partie postérieure de sa langue contre la voûte palatine, et ainsi acquérir cette nouvelle praxie.

En outre, le dispositif 2 est maintenu en bouche sans contraction de la musculature orbiculaire. Ainsi, comme ces muscles sont sensiblement au repos, le nerf facial n'est que peu ou pas stimulé lors de la déglutition. Cela permet d'apprendre une praxie dans laquelle le nerf trijumeau est le principal nerf stimulé.

L'utilisation du dispositif 2 est donc particulièrement intéressante car cet apprentissage est réalisé sans travail conscient de la part de la personne, en utilisant un câblage neurologique préexistant mais jamais stimulé. Cela signifie qu'aucun exercice particulier ni aucun geste spécifique n'est nécessaire, mis à part le port du dispositif 2.

Le procédé d'apprentissage de praxie décrit ici comprend le port par une personne du dispositif 2 pour une durée comprise entre 5 mn et 15 mn. Ce procédé doit être exercé quotidiennement sur une durée minimale d'une semaine, et jusqu'à une durée maximale de 3 mois. Le procédé peut être arrêté dès que la praxie est acquise, c'est-à-dire dès l'automatisation du geste.

L'utilisation du dispositif 2 peut être vue comme un procédé d'apprentissage d'une praxie de déglutition comprenant la sollicitation du nerf trijumeau, ainsi que d'une manière générale comme un procédé d'élargissement de la voûte palatine.

Par ailleurs, la mise en oeuvre de ce procédé ne nécessite l'intervention d'aucun personnel médical, ni de cadre thérapeutique particulier, puisqu'il suffit de mettre le dispositif en bouche.

À ce jour, ces travaux restent précurseurs dans ce domaine, et il n'existe pas de méthode ni de dispositif adapté à une telle mise en oeuvre.

Le Demandeur a mis au point un dispositif d'apprentissage de la déglutition adulte. Le dispositif est une aide à l'apprentissage de la praxie de déglutition adulte. Le dispositif est solliciteur du nerf trijumeau. Le dispositif est un modificateur d'un équilibre musculaire. D'un autre point de vue, l'invention vise un dispositif de conditionnement par de nouvelles images motrices de mouvements habituels dénués de contrôle conscient. Le dispositif est anti-succion ou anti-déglutition primaire. Par cet effet, le dispositif est aussi un outil de correction de dysmorphoses squelettiques.

Le dispositif 2 peut être prévu par exemple en trois modèles de tailles différentes, dits modèle de petite taille, modèle de taille moyenne et modèle de grande taille. De manière générale, le dispositif 2 peut prendre les dimensions suivantes :
- une largeur totale, c'est-à-dire une distance extérieure entre les extrémités 22 et 24 comprise entre 3 cm et 5,5 cm,
   - comprise entre 3,2 cm et 4 cm pour le modèle de petite taille, par exemple 3,6 cm,
   - comprise entre 3,8 cm et 4,6 cm pour le modèle de taille moyenne, par exemple 4,2 cm, et
   - comprise entre 4,5 cm et 5,5 cm pour le modèle de grande taille, par exemple 5 cm ;
- une largeur d'ouverture 26, c'est-à-dire une distance intérieure entre les extrémités 22 et 24 comprise entre 2 cm et 5 cm,
   - comprise entre 2,2 cm et 3,2 cm pour le modèle de petite taille, par exemple 2,7 cm,
   - comprise entre 2,8 cm et 3,8 cm pour le modèle de taille moyenne, par exemple 3,3 cm, et
   - comprise entre 3,3 cm et 4,3 cm pour le modèle de grande taille, par exemple 3,8 cm ;
- une hauteur d'ouverture 26, sensiblement à mi-chemin entre les deux extrémités respectives 22, 24 comprise entre 3 mm et 25 mm (supérieure à celle revendiquée),
   - comprise entre 3 mm et 8 mm pour le modèle de petite taille, par exemple 7 mm,
   - comprise entre 5 mm et 12 mm pour le modèle de taille moyenne, par exemple 10 mm, et
   - comprise entre 10 mm et 20 mm (supérieure à celle revendiquée) pour le modèle de grande taille, par exemple 15 mm ;
- une hauteur de branches 8 et 12 comprise entre 5 mm et 20 mm,
   - comprise entre 8 mm et 16 mm pour le modèle de petite taille, par exemple 12 mm,
   - comprise entre 9 mm et 17 mm pour le modèle de taille moyenne, par exemple 13 mm, et
   - comprise entre 10 mm et 18 mm pour le modèle de grande taille, par exemple 14 mm ;
- une largeur de branches 8 et 12 équivalente à la largeur totale du dispositif ou comprise entre 2 cm et 5 cm,
   - comprise entre 2 cm et 2,5 cm pour le modèle de petite taille, par exemple 2,2 cm,
   - comprise entre 3 cm et 3,5 cm pour le modèle de taille moyenne, par exemple 3,2 cm, et
   - comprise entre 4 cm et 4,5 cm pour le modèle de grande taille, par exemple 4,2 cm ;
- une profondeur des parties 16 des branches 10 et 14, c'est-à-dire la largeur de la base des gouttières, comprise entre 3 mm et 30 mm,
   - comprise entre 5 mm et 15 mm pour le modèle de petite taille, par exemple 11 mm,
   - comprise entre 8 mm et 14 mm pour le modèle de taille moyenne, par exemple 12 mm, et
   - comprise entre 12 mm et 20 mm pour le modèle de grande taille, par exemple 13 mm ; et
- une hauteur des portions recourbées 18, hors découpe 20, comprise entre 2 mm et 20 mm,
   - comprise entre 4 mm et 14 mm pour le modèle de petite taille, par exemple 9 mm,
   - comprise entre 5 mm et 15 mm pour le modèle de taille moyenne, par exemple 10 mm, et
   - comprise entre 8 mm et 18 mm pour le modèle de grande taille, par exemple 13 mm.

L'épaisseur de chacune des parties du dispositif 2 peut comprendre des parois d'épaisseur comprise entre 0,2 mm et 2 mm, par exemple 1,5 mm.

## Revendications

1. Dispositif buccal, agencé pour être porté en bouche par une personne, et pour solliciter le nerf trijumeau lors de la déglutition, comprenant une portion supérieure (4) présentant une forme sensiblement en gouttière, et propre à se loger entre une lèvre supérieure et une arcade dentaire, une portion inférieure (6) présentant une forme sensiblement en gouttière, et propre à se loger entre une lèvre inférieure et une arcade dentaire, ainsi qu'une ouverture (26) entre la portion supérieure (4) et la portion inférieure (6), et la portion supérieure (4) et la portion inférieure (6) sont reliées entre elles à des extrémités respectives (22, 24), de sorte que, lorsque le dispositif (2) est reçu dans une bouche, la musculature labiale est sensiblement au repos, et au moins une partie de l'ouverture (26) reste libre, l'ouverture (26) présentant une hauteur maximale d'environ 1,5 cm.

2. Dispositif selon la revendication 1, dans lequel la portion supérieure (4) comprend deux branches (8, 10) formant un logement pour la musculature labiale.

3. Dispositif selon la revendication 2, dans lequel la portion inférieure (6) comprend deux branches (12, 14) formant un logement pour la musculature labiale.

4. Dispositif selon la revendication 3, dans lequel la portion supérieure (4) et la portion inférieure (6) comprennent chacune une découpe (20) propre à accommoder un frein de lèvre.

5. Dispositif selon l'une des revendications 1 à 4, dans lequel l'ouverture (26) présente une forme sensiblement oblongue.

6. Dispositif selon l'une des revendications 1 à 5, dans lequel l'ouverture (26) présente une hauteur maximale comprise entre 3 mm et 1,5 cm.

7. Dispositif selon l'une des revendications 1 à 6, dans lequel l'ouverture (26) présente une largeur maximale comprise entre 2 cm et 9 cm.

8. Dispositif selon l'une des revendications 1 à 7, dans lequel le dispositif présente une largeur comprise entre 3 cm et 10 cm.

9. Dispositif selon l'une des revendications 1 à 8, dans lequel la portion supérieure (4) et la portion inférieure (6) présentent chacune une épaisseur plus importante à l'opposé de l'ouverture (26) qu'à proximité de l'ouverture (26).

## Patentansprüche

1. Orale Vorrichtung, die dazu eingerichtet ist, von einer Person im Mund getragen zu werden und beim Schlucken auf den Trigeminusnerv einzuwirken, umfassend ein Oberteil (4), das eine im Wesentlichen rinnenförmige Form aufweist und sich dazu eignet, zwischen einer Oberlippe und einer Zahnreihe zu liegen zu kommen, und ein Unterteil (6), das eine im Wesentlichen rinnenförmige Form aufweist und sich dazu eignet, zwischen einer Unterlippe und einer Zahnreihe zu liegen zu kommen, sowie eine Öffnung (26) zwischen dem Oberteil (4) und dem Unterteil (6), und wobei das Oberteil (4) und das Unterteil (6) an jeweiligen Enden (22, 24) so miteinander verbunden sind, dass, wenn die Vorrichtung (2) in einem Mund aufgenommen ist, die Labialmuskulatur im Wesentlichen im Ruhezustand ist und zumindest ein Teil der Öffnung (26) frei bleibt, wobei die Öffnung (26) eine maximale Höhe von ca. 1,5 cm aufweist.

2. Vorrichtung nach Anspruch 1, wobei das Oberteil (4) zwei Schenkel (8, 10) hat, die eine Aufnahme für die Labialmuskulatur bilden.

3. Vorrichtung nach Anspruch 2, wobei das Unterteil (6) zwei Schenkel (12, 14) hat, die eine Aufnahme für die Labialmuskulatur bilden.

4. Vorrichtung nach Anspruch 3, wobei das Oberteil (4) und das Unterteil (6) jeweils einen Ausschnitt (20) haben, der sich dazu eignet, eine Lippenbremse aufzunehmen.

5. Vorrichtung nach einem der Ansprüche 1 bis 4, wobei die Öffnung (26) eine im Wesentlichen längliche Form aufweist.

6. Vorrichtung nach einem der Ansprüche 1 bis 5, wobei die Öffnung (26) eine maximale Höhe aufweist, die zwischen 3 mm und 1,5 cm beträgt.

7. Vorrichtung nach einem der Ansprüche 1 bis 6, wobei die Öffnung (26) eine maximale Breite aufweist, die zwischen 2 cm und 9 cm beträgt.

8. Vorrichtung nach einem der Ansprüche 1 bis 7, wobei die Vorrichtung eine Breite aufweist, die zwischen 3 cm und 10 cm beträgt.

9. Vorrichtung nach einem der Ansprüche 1 bis 8, wobei das Oberteil (4) und das Unterteil (6) jeweils eine Dicke aufweisen, die gegenüber der Öffnung (26) größer ist als im Nahbereich der Öffnung (26).

## Claims

1. An oral device intended to be worn by a person in the mouth to stress the trigeminal nerve when swallowing, comprising an upper portion (4) having a substantially gutter shape and intended to be located between an upper lip and a dental arch, a lower portion (6) having a substantially gutter shape and intended to be located between a lower lip and a dental arch, and an opening (26) between the upper portion (4) and the lower portion (6), the upper portion (4) and the lower portion (6) being connected together at respective extremities (22, 24) so that when the device (2) is positioned in the mouth the labial musculature is substantially at rest and at least part of the opening (26) remains unobstructed, the opening (26) having a maximum height of about 1.5 cm.

2. A device according to claim 1, in which the upper portion (4) comprises two limbs (8, 10) forming a housing for the labial musculature.

3. A device according to claim 2, in which the lower portion (6) comprises two limbs (12, 14) forming a housing for the labial musculature.

4. A device according to claim 3, in which the upper portion (4) and the lower portion (6) each comprise a re-entrant (20) intended to accommodate a frenulum of a lip.

5. A device according to any one of claims 1 to 4, in which the opening (26) has a substantially oblong shape.

6. A device according to any one of claims 1 to 5, in which the opening (26) has a maximum height of between 3 mm and 1.5 cm.

7. A device according to any one of claims 1 to 6, in which the opening (26) has a maximum width of between 2 cm and 9 cm.

8. A device according to any one of claims 1 to 7, in which the device has a width of between 3 mm and 10 cm.

9. A device according to any one of claims 1 to 8, in which the upper portion (4) and the lower portion (6) each have a greater thickness distally from the opening (26) than proximally to the opening (26).
